# NEW EUROPEAN PATENT SPECIFICATION

(11) **EP 1 179 180 B2**
(45) Date of publication and mention of the opposition decision: **20.08.2008**
(45) Mention of the grant of the patent: 09.10.2002
(21) Application number: 00929132.9
(22) Date of filing: 16.05.2000
(51) Int. Cl.: G01N 33/543, C12Q 1/68, G01N 21/86

(54) **METHOD FOR THE IDENTIFICATION AND/OR THE QUANTIFICATION OF A TARGET COMPOUND**
VERFAHREN ZUR IDENTIFIZIERUNG UND/ODER QUANTIFIZIERUNG EINER ZIELVERBINDUNG
PROCEDE PERMETTANT L'IDENTIFICATION ET/OU LA QUANTIFICATION D'UN COMPOSE CIBLE

(30) Priority: 19.05.1999 EP 99870106; 18.02.2000 EP 00870025
(43) Date of publication of application: 13.02.2002
(73) Proprietor: Eppendorf Array Technologies, 5000 Namur (BE)
(72) Inventor: REMACLE, José, B-5020 Malonne (BE); DEMARTEAU, Joseph, B-5000 Namur (BE); ZAMMATTEO, Nathalie, B-5100 Jambes (BE); ALEXANDRE, Isabelle, B-5170 Lesve (BE); HAMELS, Sandrine, B-6280 Loverval (BE); HOUBION, Yves, B-5150 Floreffe (BE); DE LONGUEVILLE, Françoise, B-5100 Jambes (BE)
(74) Representative: Ricker, Mathias
(86) International application number: PCT/BE2000/000054
(87) International publication number: WO 2000/072018

(56) References cited:
- EP-A- 0 646 784
- WO-A-94/19767
- WO-A-95/11995
- WO-A-97/10365
- WO-A-99/35499
- US-A- 4 244 797
- US-A- 4 244 803
- US-A- 5 270 167
- Chen et al.(1998). Genomics, 51, 313-324

## Description

### Field of the invention

The present invention is related to a method for the identification and/or the quantification of a target compound obtained from a biological sample by binding to a capture molecule fixed upon chips.

The present invention is also related to an identification and/or quantification apparatus based upon said method, that allows the identification and/or the quantification of positive locations of bounded target compounds upon said chips.

### Background on the invention and state of the art

Biological assays are mainly based upon interaction specificity between two biological molecules such two strands of nucleic acid molecules, an antigen and an antibody or a ligand and its receptor. The present challenge of biological assays is to perform simultaneously the multiple detection of molecules present in a sample. Miniaturisation and development of arrays upon the surface of "biochips" are tools that allow multiplex reactions in a microscopic format, said detection being made with a limited volume of sample for the screening and/or the identification of multiple possible target compounds. These arrays are formed of discrete regions, containing a specific capture molecule used for the binding of the target compound. These discrete regions, as small as a few micrometers, allow the fixation of several thousands capture molecules per cm² surface (WO 95/11995).

However, the detection of bounded target compounds is difficult, since their amount is very small due to said miniaturisation (few fentomoles or even few attomoles). Therefore, only extremely sensitive methods are adequate for such detection.

It has been proposed a labelling of a target compound like DNA with fluorescent molecules after their possible genetic amplification. When an RNA molecule has to be detected, it is first transformed into a cDNA, before its possible amplification. If direct labelling of the target compound is not possible, a double reaction (sandwich reaction) can be performed. However, the amount of fluorescent molecules is so low that it is necessary to develop specific array scanners for the detection and/or the quantification of the bounded compound upon the "hybridisation chips". Said expensive specific scanners comprise a laser scanner for excitation of the fluorescent molecules, a pinhole for decreasing the noise fluorescent background, and a photomultiplier for increasing the sensitivity of the detection.

Alternative detection methods that present a high sensitivity are described in the documents US-5,821,060 and WO95/04 and are based upon the detection using expensive devices such as mass spectrometers.

It has also been proposed methods based upon the precipitation of specific products resulting of a colorimetric labelling (US 5,270,167, US 4,731,325, EP-A-0301141), the result of an enzymatic activity (EP-A-0393868, WO 86/02733, EP-A-0063810) or obtained by immunodiffusion and immunoelectrophoresis (US 4, 244, 797 ; US 4, 244, 803). However, said methods are either characterized by a low sensitivity or are not adequate for the detection of a target compound upon "hybridisation chips", because the precipitate will occur at a certain distance of the reaction binding and its location can not be easily correlated with a specific bounded target compound. In addition, the density of the precipitate of such enzymatic reactions is not enough opaque for allowing a detection by light absorption.

It has also been proposed to improve the detection by fixing a soluble product obtained from the enzymatic reaction with a metal before its precipitation. However, as the result of said enzymatic reaction is a soluble product, there is no correlation between the location of the precipitate and the detection of a specific bounded target compound.

### Aims of the invention

The present invention aims to provide a new identification and/or quantification method of one or more target compounds present (possibly simultaneously) in a biological sample and that will not present the drawbacks of the state of the art.

The present invention aims to provide such a method that is simple and not expensive, that allows the detection of said target compounds by using fixed capture molecules upon arrays of the surface of a solid support.

A last aim of the present invention is to provide also a simple and non-expensive apparatus based upon said method, that improves the identification and/or the quantification of bounded target compounds upon "hybridisation chips".

### Summary of the invention

The present invention is related to a method for identification and/or quantification of at least one target compound present in a biological sample by its binding upon a capture molecule fixed upon arrays of a solid support (hereafter called "hybridisation chips"), the binding of said target compound upon its corresponding capture molecule resulting in the formation of a metal precipitate at the location of said capture molecule.

As recited in claim 1, the present invention relates to a method for the identification and/or the quantification of a target compound obtained from a sample, preferably a biological sample, comprising the steps of:
- putting into contact the target compound with a capture molecule in order to allow a specific binding between said target compound with a capture molecule, said capture molecule being fixed upon a surface of a solid support according to an array comprising a density of at least. 20 discrete regions per cm^{2,} each of said discrete regions being fixed with one species of capture molecules,
- performing a reaction leading to the formation of a precipitate formed at the location of said binding and obtained by the precipitate of a metallic compound upon the bounded target compound,
- determining the possible formation and/or presence of precipitate(s) in discrete region(s), and correlating the formation and/or presence of the precipitate(s) at.the discrete region(s) with the identification and/or a quantification of said target compound, under the proviso that as a solid support a compact disc, wherein the non-cleavable capture molecules on the external surface of the compact disc are addressed by conventional physical methods using microlithographic and / or micromachining techniques of incubation which will maintain the non-cleavable capture molecules at certain locations where they will be fixed, is excluded.

Preferably, said method comprises the further steps of:
- correlating the presence of the metallic precipitate(s) at the discrete region(s) (precipitate pattern) with the identification and/or a quantification of said target compound in the biological sample.

The "hybridisation chips" according to the invention are any kind of solid support that allow the formation of arrays of capture molecules (specific pattern) upon one or more of its surfaces. Said solid support can be made of glasses, filters, electronic device, polymeric or metallic materials, etc. Preferably, said arrays contain specific locations (advantageously presented according to a specific pattern), each of them containing normally only one species of capture molecule.

The fixation of DNA strands on proteins thereafter specifically attached to sites specific locations on a substrate, is described in the document US-5,561,071. It is also known that capture chemicals can be linked to microtubes that are then spatially arranged in order to produce an array, as described in the document GB-3 319 838, or to obtain the direct synthesis of oligonucleotides on specific surfaces by using photolithographic techniques as described in the documents EP-0476014, US-5,510,270, US-5,445,934, WO97/29212, US-5,605,662, US-5,632,957 and WO94/22889.

All these methods for the fixation of capture molecules on the surface of a solid support in order to obtain the above-described arrays are compatible with the present invention.

The biological target compounds according to the invention may be present in a biological (or possibly a non-biological) sample such as clinical samples extracted from blood, urine, faeces, saliva, pus, serum, tissues, fermentation solutions or culture media. Said target compounds are preferably isolated purified, cleaved, copied and/or amplified if necessary by known methods by the person skilled in the art before their detection and/or quantification upon the "hybridisation chips".

Preferably, the formation of a metallic precipitate at the location of the binding is obtained with the fixation of a metallic compound upon the bounded target compound or by the result of a reduction of a metal in the presence of an enzyme. Advantageously, a reduction of silver in the presence of colloidal gold allows the formation of a precipitate at a distance not exceeding few micrometers from the bounded target compound to its capture molecule.

According to the invention, the specific locations on the array are smaller than 1000 µm in length. These locations or spots have preferably a diameter comprised between 10 and 500 µm and are separated by distance of similar order of magnitude, so that the array of the solid support comprises between 100 and 250,000 spots upon the surface of 1 cm². However, it is also possible to prepare spots smaller as 1 µm or less upon which the capture molecules are fixed. The formation of said spots or locations would be obtained by known microelectronic or photolitographic processes and devices that allow the fixation of said capture molecules on the surface of the solid support either by a covalent linkage or a non-covalent adsorption. The covalent linkage technique is preferred in order to control specifically the sites of capture molecules fixation and avoid possible drawbacks that may result with several capture molecules (like nucleic acids or antibodies) that can be desorbed during incubation or washing step.

One of the preferred embodiment is the fixation of biological molecules like proteins, peptides or nucleic acid sequences by linkage of amino groups on activated glass bearing aldehyde moiety. The incorporation of an amine group in the nucleic acid chain is easily obtained using aminated nucleotide during their synthesis. Aminated amino acids can be fixed upon the surface of a solid support like glass bearing aldehyde groups as described by Schena et al. (Proc. Natl. Acad. Sci. USA, 93, pp. 10614-10619 (1996)) or as described in the document US-5,605,662 and the publication of Krensky et al. (Nucleic Acids Research, 15, pp. 2891-2909 (1987)). The linkage between an amino and a carboxyl group is obtained by the presence of a coupling agent like carbodiimide compounds as described by Joos et al. (Anal. Biochem., 247, pp. 96-101 (1997)). Thiol modified oligonucleotides can be used also to obtain a reaction with amino groups upon the surface of a solid support in the presence of cross-linking molecules (Thrisey et al., Nucleic Acids Research, 24, pp. 3031-3039 (1996)). Similarly, oligonucleotides can be fixed to a gel like polyacrylamide bearing hydroxyl and aldehyde groups as described in the document US-5,552,270 and WO98/28444.

The binding (or recognition) of the target compound upon their corresponding specific capture molecules is a spontaneous non-covalent reaction when performed in optimal conditions. It involves non-covalent chemical bindings. The medium composition and other physical and chemical factors affect the rate and the strength of the binding. For example for nucleotide strand recognition, low stringency and high temperature lower the rate and the strength of the binding between the two complementary strands. However, they also very much lower the non specific binding between two strands (which are not perfectly complementary). When several sequences are similar, the specificity of the binding can be enhanced by addition of a small amount of non-labelled molecules, which will compete with their complementary sequence, but much more with the other ones, thus lowering the level of cross-reactions.

The optimisation of the binding conditions is also necessary for antigen/antibody or ligand/receptors recognition, but they are usually rather specific.

A preferred embodiment of this invention is to take party of the amplification given by the catalytic reduction of Ag⁺ in the contact of other metals like gold. Gold nanoparticules are currently available and they can be easily fixed to molecules like protein. For example, streptavidin coated gold particles are available on the market.

According to a preferred embodiment of this invention, one uses a labelled target molecule, which is then recognised by a conjugate. This labelled molecule (biotin, haptens, ...) can be considered as a first member of the binding pair. For DNA, the labelling is easily done by incorporation of biotinylated nucleotides during their amplification. For the RNA, biotinylated nucleotides are used for their copy in cDNA or thereafter in the amplification step. Amplification of the nucleotide sequences is a common practice since the target molecules are often present in very low concentrations. Proteins are easily labelled using NHS-biotin or other reactions. Once the biotinylated molecules are captured, a streptavidingold complex, which is the second member of the binding pair, is added and the streptavidin specifically recognises biotin, so that the complex is fixed at the location where the target is fixed. If haptens are used as label, an antibody-gold complex will be used. Then a reactive mixture containing Ag⁺ and a reducing agent is added on the surface and Ag layers will precipitate on the gold particles leading to the formation of crystal particles.

Direct labelling of the target molecules with gold is possible by using gold-labelled antigens, antibodies or nucleotides.

An alternative is to avoid any labelling of the target molecule, and then a second nucleotide sequence is used which is labelled. They then formed a sandwich hybridisation or a sandwich reaction with the capture molecule fixing the target and the labelled nucleotide sequence, which allows the detection to go on. Like above, the labelled nucleotide sequence is able to catalyse itself the precipitation of the metal or it does it through a second complex.

The Ag precipitation corresponds to the location of the binding of biotinylated nucleotide sequence. As said location is well defined, it is possible to identify the presence of said precipitate (specific spot of the array).

The metallic precipitate has the form of small crystals that reach with time a diameter of about 1 µm. The formation of these small crystals represents a real amplification of the signal since they originated from the presence of gold particles a few nm in diameter.

Unexpectedly, within a given range of labelled nucleotide sequences present on the surface, a concentration curve could be obtained between the gold-labelled nucleotide sequence concentration and the amount of precipitate on the surface. One constraint of the array is that the detection signal has to be correlated with the location where it originates.

Because of its granular form, the precipitate advantageously modifies the reflection of the light. It also leads to a strong diffusion of the light (spot detected), which is recordable by known detection means. Such diffusion assays are typically detected and recorded from the reflection of a light beam with photodiodes. One unexpected observation is that this assay for the presence of silver crystals was found unexpectedly very sensitive.

The fact that the silver precipitate appears as a black surface allows the use of a scanner (absorption of the light through the transparent surface of the array). The presence of insoluble precipitate will absorb the light, which is then detected and recorded. The advantage of the scanner is that only a small portion of the array is detected at one time so that a much better resolution can be obtained. Either the illumination beam or the detection surface is focalised and the signal is recorded so that the image of the array can be reconstituted. The detection means (detector) can be a CCD or CMOS camera, which measures the overall array. The resolution of the detection is then dependent on the number of pixels of the camera. On the other end, the detector can be constituted of photodiodes arranged into a line and the image is scanned by moving in front of this line. Scanners with a sensitivity of 11 µm for a pixel can be constructed, which are sufficient to analyse spots of 50 µm in diameter or bigger.

A full illumination of the array combined with a recording of the light transmitted is also possible (faster than the scanner, but seems to be less sensitive).

As a metal, silver is able to reflect light by itself. Even if the efficiency of this reflection is low, it exists and can be used in order to localise the silver precipitate (spots). Because if its metal nature, other methods like variations of an electromagnetic field or electric conductance are also possible.

Another aspect of the present invention concerns a diagnostic and/or quantification apparatus of one or more identical or different target compound(s) obtained from a sample, said apparatus comprising:
- capture molecules being fixed upon a surface of a solid support according to an array comprising a density of at least 20 discrete regions per cm², each of said discrete regions being fixed with one species of capture molecule,
- a metallic precipitate formed at a location of the binding between said target compound with said capture molecule,
- a detection and/or quantification device of a metallic precipitate formed at a location of the binding between said target compound with said capture molecule,
- possibly a reading device of information(s) recorded upon said solid support, and
- a computer programmed to:
   - possibly recognize discrete regions bearing capture molecules,
   - collect the results (resulting from the formation of a metallic precipitate at a specific location) obtained from said detection device, and possibly the information(s) obtained from said reading device, and
   - carry out a diagnostic and/or quantification of said target compound(s), under the proviso that as a solid support a compact disc, wherein the non-cleavable capture molecules on the external surface of the compact disc are addressed by conventional physical methods using microlithographic and / or micromachining techniques of incubation which will maintain the non-cleavable capture molecules at certain locations where they will be fixed, is excluded.

Hence, detection resolution, and more particularly the reliability of the final quantification depends largely on the characteristics of the detection device. Especially, when the detection device includes a CCD camera, the reliability depends on its number of pixels. The number of pixels thus limits the allowed sensitivity of the quantification. Typically, it is possible to obtain with a CCD a resolution of 10 µm for a pixel, which are sufficient to analyse spots of 100 µm in diameter or bigger. However, such quantification is limited by the number of pixels, by the resolution of each pixel and the fact that the sensitivity is given by only one view point. One view point depends on the three following patterns : the position of the lecture element like CCD camera, the position of the object to be detected and the position of the lightening of the object.

In order to respond to said objectives, the present invention is also related to a method (dedicated to the detection and/or the quantification of a precipitate according to the invention, metallic) for the quantification of a volume of a precipitate (metallic) upon a defined surface of a solid support, said defined surface of a solid support being defined by an array of at least 4, at least 10, at least 16, at least 20 or more discrete regions per cm², each discrete region possibly comprising a precipitate. According to the invention images of said defined surface comprising one or more precipitates correspond to different views, said images containing analogue informations being taken by one or several camera(s) and upon illumination by one or several illuminant source(s) being spatially arranged relatively to each other according to a predetermined pattern; the corresponding image analogue informations of said defined surface comprising said precipitate(s) being transformed and converted into digital form or set of digital forms and compared to a first and to a second reference standard to determine the volume of the precipitate(s) to be quantified.

The first reference standard corresponds to the digital form or set of digital forms obtained from analogue informations contained in images taken on said surface without metallic precipitate.

The second reference standard corresponds to the digital form or set of digital forms obtained from analogue informations contained in images taken on said surface comprising metallic precipitates of known volume.

The term "volume" should be understood to mean the volume for which it is desired to obtain dimensional-type information. In the present invention, said volume results from a chemical or biochemical reaction following a binding between a target compound and its corresponding compound. Therefore, said obtained volume is the expression of said chemical or biochemical reaction following a binding between a target compound and its corresponding capture compound.

The term "image" should be understood to mean a group of pixels which is an illustration of a measure of said volume and which may be directly transmitted to and registered upon a monitor such as a screen or a printer.

The present invention is also related to an apparatus comprising means for implementing said method, preferably comprising one or several sensor(s) provided with cameras and with one and/or several illuminant source(s) which are spatially arranged relatively to each other according to a predetermined pattern and which are associated with an analogue information acquisition system, the information being measured by using said sensors and being converted into digital form by a processing unit.

Preferably the transformation and conversion are made by a processing unit on board of the camera or in a computer.

The cameras are preferably mono, infrared, colour, special adjacent range CCD or CMOS cameras or similar lecture technologies.

The illuminant source is preferably an infrared light having a wavelength similar to the dimension of the metal crystals contained in the precipitate(s) and which is advantageously produced by using a single diode or diodes having the same spectral distribution.

The illuminant sources are advantageously regularly spaced around the solid support, each of said sources corresponding to a light spot, which can be automatically switched on simultaneously or successively.

The images are preferably obtained either by transparency, by reflection or by a combination thereof.

As illustrated in the enclosed drawings, the apparatus and method may comprise the use of one camera and one illuminant source, placed above the solid support, said camera and said illuminant source being movable in the three dimensions in space.

The apparatus and method may comprise also the use of two or more cameras oppositely arranged in a plane and placed above the solid support and one or more illuminant sources placed under the solid support.

The apparatus and method may comprise also the use of three or more cameras arranged according to a triangular plane or another regular or irregular pattern and placed above the solid support and one or more illuminant sources placed under the solid support.

The apparatus and method may comprise the use of one camera placed above the solid support, a first illuminant source placed above the solid support and under said camera, a second illuminant source placed under the solid support, the two illuminant sources being placed almost symmetrically according to the position of the solid support.

Alternative preferred embodiments of the present invention are based upon the use of one or more illuminant source(s) and one or more camera(s) which may be used according to the method of the present invention, either in combination or consecutively, said illuminant source(s) and/or said camera(s) can be maintained fixed during the lecture or can be moved according to a preferred translation or rotation movement along or around the solid support comprising the specific volume of a precipitate.

It is also possible by using one or more illuminant source(s) and/or one or more camera(s) to allow the movement of the solid support comprising a specific volume of a precipitate.

Other embodiments that may be used according to the invention are apparatus comprising (i) either one camera and several illuminant sources with the different illuminant sources arranged from each other according to different symmetric or non-symmetric patterns, (ii) or one illuminant source and several cameras, said cameras being arranged from each other according to different symmetric or non-symmetric patterns, (iii) or a combination thereof. The illuminant is an infra-red light having a wavelength similar to the dimension of the crystals contained in the precipitate.

The person skilled in the art is also able to provide means for performing the various steps of the present invention, especially the transformation and the conversion of the major volume into digital form or a set of digital forms by using known means or methods such as the ones present in the software and computer technologies.

Said means are able to collect the results obtained from said detection and/or quantification device and possibly the information(s) obtained by said reading device, and said means are able to carry out a diagnostic and/or quantification of a specific target compound resulting from the analysis of said results, possibly correlated to the read information(s).

Said means of this computer program product are able to obtain a discrimination between the spots and a possible detected background noise, for instance by the identification of homogeneous parts of an image after having been merged into two classes used as training sets. This discrimination can be enhanced by post-classification contextual filters techniques.

Said means are also able to identify the contour of the spot itself, which will be superposed to the original image and will allow the measure of intensity level of the counted pixels identified in the spot.

The quantification means allow an integration of all pixels intensity present in the spot or a recording the overall level of intensity of the homogeneous parts of the spot.

Furthermore, these means allow a statistical comparative analysis between the spots of each sample and a control or reference standard (standard target compound) or between two or more spots (preferably with a correlation with the recorded information of the solid support). Image correlation could be obtained between the spot image and said standard target compound spot image in order to discriminate spots that are statistically different in one test compared to another.

The recorded signal(s) by the detection device and the reading device can be read, processed as electronically computerised data, analysed by said appropriate computer program product (software).

According to a specific embodiment of the present invention, the array bears fixed oligonucleotide capture nucleotide sequences so as to allow a detection, amplification and possibility quantification of nucleic acid sequences upon a same solid support. In an alternative form of execution, the array comprises fixed PCR primers in order to obtain the production of amplicons and fixation of amplicons upon the surface according to the method described by Rasmussen et al. (Anal. Biochem., 198, pp. 138-205 (1991)), which allows thereafter their detection.

The array according to this invention is used in a diagnostic and/or quantification apparatus which allows automatic lecture, possibly after a previous treatment, such as purification, cleaving, copying and/or genetic amplification.

Preferably, the detection and/or quantification apparatus according to the invention is a system that combines multiple steps or substeps within an integrated system as an automatic nucleic acid diagnostic system (the steps of purification of the nucleic acid sequences in a sample, of amplification (through known genetic amplification methods), the diagnostic and possibly the quantification).

Preferred embodiments of the present invention will be described in the following non-limiting examples in reference to the figures.

### Short description of the drawings

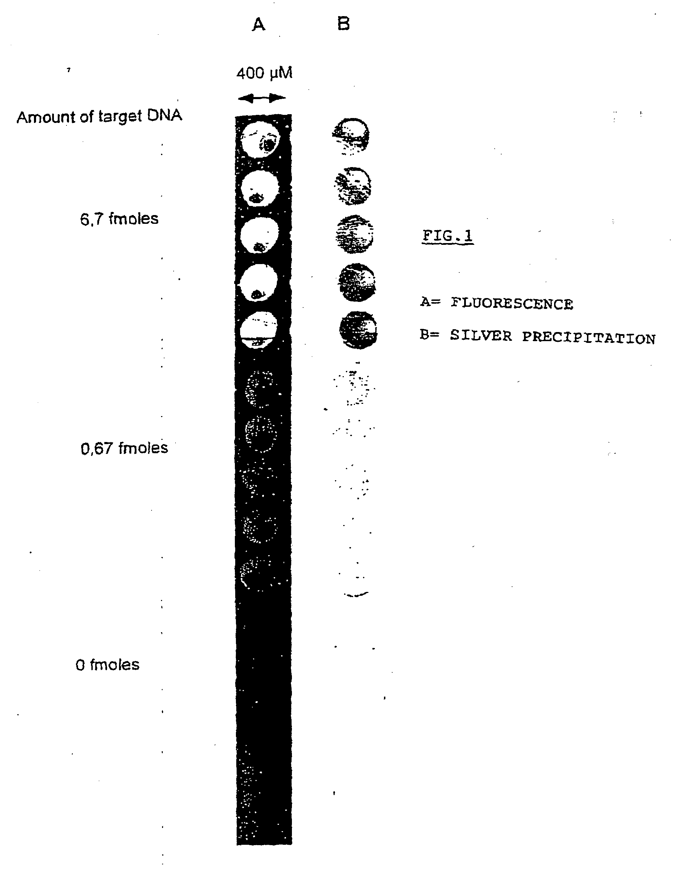

The figure 1 compares the detection of target molecules obtained on arrays composed of DNA capture nucleotide sequences covalently fixed on glass and used to detect 3 concentrations of biotinylated target DNA either in fluorescence or after silver concentration.

The figures 2 to 7 represent the spatail arrangement of some elements in various embodiments of the apparatus for performing the detection and/or the quantification method according to the invention.

### Example 1

### Detection of DNA on biochips

In this experiment, target DNA labelled is detected by direct hybridisation on capture nucleotide sequences bound to the array. Capture nucleotide sequences were covalently bound on glass and direct hybridisation performed with complementary biotinylated DNA. The positive hybridisation was detected with silver precipitate catalysed by the nanogold particles linked to streptavidin.

### Binding of capture nucleotide sequences on glass

Activated glass bearing aldehyde groups were purchased from CEL Associates (USA). Aminated capture nucleotide sequences for CMV DNA were constructed by PCR amplification of the DNA using aminated primer as described by Zammatteo et al. (Anal. Biochem., 253, pp. 180-189 (1997)). The primers were purchased from Eurogentec (Liège, Belgium). Quantification of the amplicons was done by their absorption at 260 nm.

For the grafting on glass, a solution of aminated amplicons at 0.2 µm in MES 0.1 M pH 6.5 was first heated at 100 °C for 5 min and then spotted by a robot using 250 µm diameter pins (Genetix, UK). After incubation of 1 h at 20 °C, they were washed with SDS solution at 0.1% and then two times with water. They were then incubated with NaBH₄ at 2.5 mg/ml solution for 5 min then washed in water and heated at 95 °C for 3 min before being dried.

### Hybridisation of the target molecule

The target molecule was obtained by amplification by PCR in the presence of biotinylated dUTP at 1 mM (Alexandre et al., Biotechniques, 25, pp. 676-683 (1998)). Plasmids containing the sequence of CMV virus were used for the PCR. After amplification, the PCR products were purified using a kit of high pure PCR product purification (Boehringer, Mannheim, Germany) and quantified by ethidium bromide staining after separation on a 2% agarose gel.

For the hybridisation, various concentrations 0.67, 6.7 and 67 fm in 5 µl of biotinylated target DNA were added in a SSC 2X Denhard solution containing 20 µg of Salmon DNA. A drop of this solution (5 µl) was added on the array and incubated for 2 h at 65 °C in a wet atmosphere. The array was then washed 4 times with a maleic acid buffer 10 mM pH 7.5, containing NaCl 15 mM and Tween 0.1%.

### Silver precipitation on the array after silver precipitation

The array was first incubated for 45 min with 0.8 ml of a streptavidin-colloidal gold (Sigma) diluted 1,000 times in a maleic buffer 150 mM pH 7.4 containing NaCl 100 mM and 0.1% dry milk ponder. The arrays were then washed 5 times 2 min in the maleic acid buffer 10 mM pH 7.4 containing 15 mM NaCl and Tween 0.1%. A "silver enhancement reagent" (40 µl) from Sigma was added onto the array and changed after 10 and then 5 min. After washing in the maleic buffer, the array was dried.

### Detection and analysis of the array

The array was scanned and the digitalised image was treated with form recognition software in order to delimitate and identify the spots. The level of the pixels of each spot was integrated and a value given to each spot. The values were corrected for the background obtained in three places where no capture nucleotide sequences have been fixed.

### Example 2

### Detection of proteins on biochips

### Fixation of antibodies on the array

The glass of the array was activated as described here above in order to obtain aldehyde groups on the surface. The antibodies used in this experiment were raised against bovine serum albumin for positive control and non specific IgG for negative control. The antibodies at 10 µg/ml in PBS solution were spotted using the 250 µm diameter pins directly on the glass. The amino groups of the antibodies could react with the aldehyde present on the glass. The reaction was performed for 1h at room temperature. The glasses were washed with a PBS buffer.

### Detection of bovine serum albumin by ELISA on the array

A solution of bovine serum albumin (BSA) at 10 µg/ml in PBS containing 0.1% casein was added on the array and incubated for 30 min. The array was then washed 3 times with, PBS containing 0.1% Tween 20 and then incubated with a solution of biotinylated anti-BSA at 20 µg/ml in PBS containing 0.1% casein. The incubation was performed for 30 min. A streptavidin-Gold complex at 1 µg/ml was then incubated for 30 min in a PBS solution containing 0.1% casein. The presence of gold served as a center for silver reduction. The silver precipitation was performed with a "silver enhancement reagent" from Sigma with a change of the solution after 10 min and then again after 5 min. The glasses were then scanned and the data analysed as presented in the example here above.

### Example 3

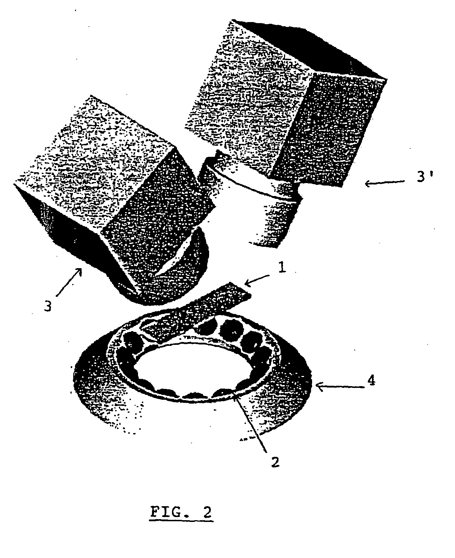

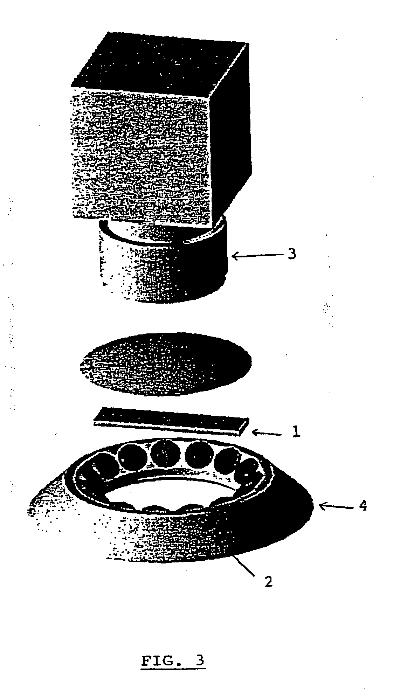

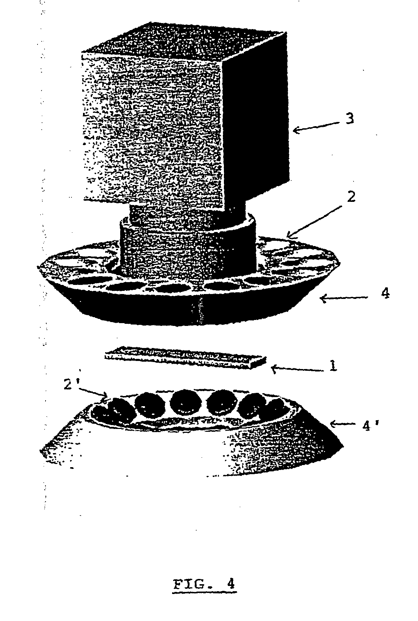

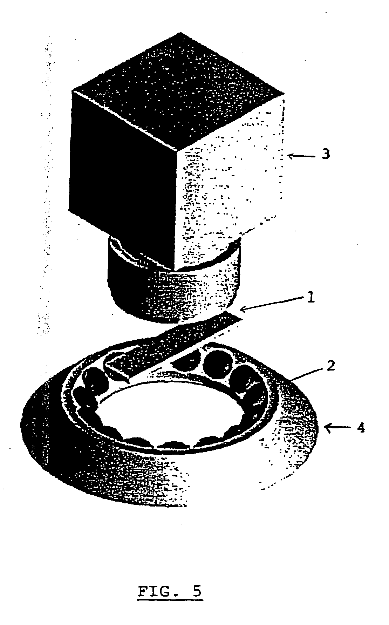

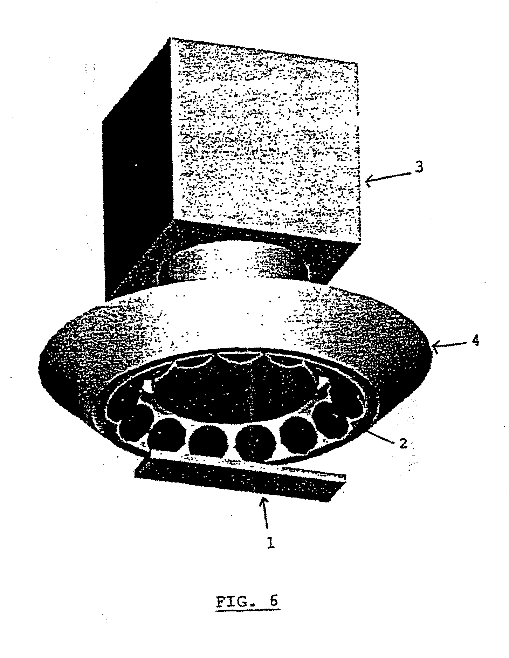

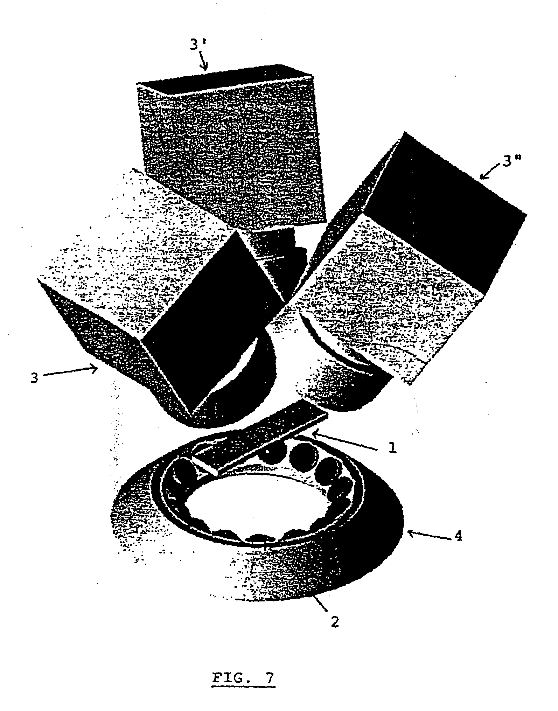

Preferred embodiments of the apparatus for performing the quantification method according to the invention is shown in the figures 2 to 7. The apparatus comprises a solid support 1, several illuminant sources 2 regularly spaced from each other on a circular support 4, said circular support being placed under said solid support 1, and two cameras 3, 3', said cameras being placed above said solid support 1 and being arranged oppositely in a plane.

The apparatus may also comprise a solid support 1, several illuminant sources 2 regularly spaced from each other on a circular support 4, said circular support being placed under said solid support 1, and one camera 3 placed above said solid support 1.

Further, the apparatus may also comprise a solid support 1. The apparatus comprises also a first set of illuminant sources 2 and a second set of illuminant sources 2, the illuminant sources of each set 2, 2' being regularly spaced from each other in a plane, preferably on a circular support 4, 4'. The first set of illuminant sources 2 is placed above the solid support 1 and the second set 2' is placed under said solid support 1, said first and said second sets of illuminant sources 2, 2' being placed symmetrically according to the position of said solid support 1. The apparatus also comprises a camera 3 placed above said solid support 1 and above the first set of illuminant sources 2.

Further, the apparatus may also comprise a solid support 1, with or without several illuminant sources 2 being regularly spaced from each other in a plane, preferably on a circular support 4, and being placed under the solid support 1. The apparatus comprises also a camera 3 placed above. Said circular support 4 and said camera 3 are placed symmetrically according to the position of the solid support 1.

Finally, the apparatus may also comprise a solid support 1 with several illuminant sources 2 regularly spaced from each other in a plane, preferably on a circular support 4, said circular support being placed under said solid support 1, and three cameras 3, 3', 3'', said cameras being placed above said solid support 1 and being arranged according to triangular arrangement in a plane.

## Claims

1. A method for the identification and/or the quantification of a target compound obtained from a sample, preferably a biological sample, comprising the steps of:
- putting into contact the target compound with a capture molecule in order to allow a specific binding between said target compound with a capture molecule, said capture molecule being fixed upon a surface of a solid support according to an array comprising a density of at least 20 discrete regions per cm², each of said discrete regions being fixed with one species of capture molecules,
- performing a reaction leading to the formation of a precipitate formed at the location of said binding and obtained by the precipitate of a metallic compound upon the bounded target compound,
- determining the possible formation and/or presence of precipitate(s) in discrete region(s), and correlating the formation and/or presence of the precipitate(s) at the discrete region(s) with the identification and/or a quantification of said target compound, under the proviso that as a solid support a compact disc, wherein the non-cleavable capture molecules on the external surface of the compact disc are addressed by conventional physical methods using microlithographic and / or micromachining techniques of incubation which will maintain the non-cleavable capture molecules at certain locations where they will be fixed, is excluded.

2. The method according to claim 1, **characterized in that** said metallic compound is a magnetic metallic compound.

3. The method according to claim 1 or 2, **characterized in that** the reaction leading to the formation of the precipitate is a reduction of a metal in the presence of an enzyme.

4. The method according to claim 1 or 2, **characterized in that** the reaction leading to the formation of the precipitate is a chemical reduction of silver in the presence of colloidal gold particles coupled to the bounded target compound.

5. The method according to any one of the preceding claims, **characterized in that** the specific binding between the target compound and its corresponding capture molecule is an hybridisation between two nucleotide sequences.

6. The method according to any one of the claims 1 to 4, **characterized in that** the binding between the target compound and its corresponding capture molecule is a reaction between an antigenic structure and its corresponding antibody or a hypervariable portion thereof.

7. The method according to any one of the preceding claims, **characterized in that** the binding between the target compound and its corresponding capture molecule is a reaction between a receptor and its corresponding ligand.

8. The method according to any one of the preceding claims, **characterized in that** the possible presence of a precipitate is obtained by reflection, absorption or diffusion of a light beam, preferably a laser beam, upon said precipitate.

9. The method according to any one of the preceding claims, **characterized in that** the presence of a precipitate in a discrete region is obtained by variation of an electromagnetic field or the conductance of an electric current.

10. The method according to any of the preceding claims 1 to 9, for the quantification of volume of one or more precipitate(s) upon a defined surface of the solid support, **characterized in that** images of said defined surface containing one or more precipitate(s) and corresponding to different views, said images containing analogue information, are taken by one or more camera(s) upon illumination by one or more illuminant source(s), spatially arranged relatively to each other according to a predetermined pattern and **characterized in that** the corresponding image analogue information of said defined surface containing said precipitate(s) are transformed and converted into digital form or set of digital forms and compared to a first and to a second reference standards to determine the volume of the precipitate(s) to be quantified.

11. The method according to claim 10, **characterized in that** the first reference standard corresponds to a digital form or a set of digital forms obtained from analogue information contained in images taken on the surface of said solid support (1) without precipitate.

12. The method according to the claim 11, **characterized in that** the second reference standard corresponds to a digital form or a set of digital forms obtained from analogue information contained in images taken on the surface of said solid support (1) containing precipitate(s) of known volume.

13. Diagnostic and/or quantification apparatus of one or more identical target compound(s) obtained from a sample, which comprises:
- capture molecules being fixed upon a surface of a solid support according to an array comprising a density of at least 20 discrete regions per cm², each of said discrete regions being fixed with one species of capture molecule,
- a metallic precipitate formed at a location of the binding between said target compound with said capture molecule,
- a detection and/or quantification device of a metallic precipitate formed at a location of the binding between said target compound with said capture molecule,
- possibly a reading device of information(s) recorded upon said solid support, and
- a computer programmed to:
- possibly recognize discrete regions bearing capture molecules,
- collect the results (resulting from the formation of a metallic precipitate at a specific location) obtained from said detection device, and possibly the information(s) obtained from said reading device, and
- carry out a diagnostic and/or quantification of said target compound(s), under the proviso that as a solid support a compact disc, wherein the non-cleavable capture molecules on the external surface of the compact disc are addressed by conventional physical methods using microlithographic and / or micromachining techniques of incubation which will maintain the non-cleavable capture molecules at certain locations where they will be fixed, is excluded.

## Patentansprüche

1. Verfahren zur Identifizierung und/oder Quantifizierung einer Zielverbindung, die aus einer Probe erhalten wird, vorzugsweise aus einer biologischen Probe, wobei das Verfahren folgende Schritte umfasst:
- das Inkontaktbringen der Zielverbindung mit einem Einfangmolekül, um eine spezifische Bindung zwischen der besagten Zielverbindung mit einem Einfangmolekül zu ermöglichen, wobei das Einfangmolekül auf eine Oberfläche eines festen Trägers gebunden wird, in einer Gruppierung, die eine Dichte von mindestens 20 diskreten Bereichen pro cm² aufweist, wobei ein jeder dieser diskreten Bereiche mit einer Spezies eines Einfangmoleküls verbunden wird;
- die Durchführung einer Reaktion, die zu der Bildung eines Niederschlages führt, der an der Stelle der Bindung gebildet wird und der durch den Niederschlag einer metallischen Verbindung über der gebundenen Zielverbindung erzielt wird,
- die Bestimmung der möglichen Bildung und / oder des Vorliegens eines oder mehrerer Niederschläge in einem oder mehreren diskreten Bereichen, und das Korrelieren der Bildung und / oder des Vorliegens eines oder mehrerer Niederschläge an einem oder mehreren diskreten Bereichen mit der Identifizierung und / oder der Quantifizierung der besagten Zielverbindung,
mit der Maßgabe, dass als fester Träger eine Kompaktdiskette (CD) ausgeschlossen wird, wobei die nicht-spaltbaren Einfangmoleküle auf der äußeren Oberfläche der Kompaktdiskette durch konventionelle physikalische Verfahren adressiert werden unter Verwendung mikrolithographischer und/oder mikroverarbeitender Techniken der Inkubation, welche die nicht-spaltbaren Einfangmoleküle an bestimmten Stellen halten, an denen sie fixiert sind.

2. Verfahren nach Anspruch 1, **dadurch gekennzeichnet, dass** die metallische Verbindung eine magnetische metallische Verbindung ist.

3. Verfahren nach Anspruch 1 oder 2, **dadurch gekennzeichnet, dass** die Reaktion, die zu der Bildung des Niederschlages führt, eine Reduktion eines Metalls in Anwesenheit eines Enzyms ist.

4. Verfahren nach Anspruch 1 oder 2, **dadurch gekennzeichnet, dass** die Reaktion, die zu der Bildung des Niederschlages führt, eine chemische Reduktion von Silber in Anwesenheit von kolloidalen Goldpartikeln ist, die an die gebundene Zielverbindung gekoppelt sind.

5. Verfahren nach irgendeinem der vorhergehenden Ansprüche, **dadurch gekennzeichnet, dass** die spezifische Bindung zwischen der Zielverbindung und ihrem entsprechenden Einfangmolekül eine Hybridisierung zwischen zwei Nukleotidsequenzen ist.

6. Verfahren nach irgendeinem der Ansprüche 1 bis 4, **dadurch gekennzeichnet, dass** die Bindung zwischen der Zielverbindung und ihrem entsprechenden Einfangmolekül eine Reaktion zwischen einer antigenen Struktur und ihrem entsprechenden Antikörper oder einem hypervariablen desselben ist.

7. Verfahren nach irgendeinem der vorhergehenden Ansprüche, **dadurch gekennzeichnet, dass** die Bindung zwischen der Zielverbindung und ihrem entsprechenden Einfangmolekül eine Reaktion zwischen einem Rezeptor und seinem entsprechenden Liganden ist.

8. Verfahren nach irgendeinem der vorhergehenden Ansprüche, **dadurch gekennzeichnet, dass** das mögliche Vorliegen eines Niederschlages durch Reflektion, Absorption oder Diffusion eines Lichtstrahles, vorzugsweise eines Laserstrahles, an dem besagten Niederschlag erzielt wird.

9. Verfahren nach irgendeinem der vorhergehenden Ansprüche, **dadurch gekennzeichnet, dass** das Vorliegen eines Niederschlages in einem diskreten Feld durch Veränderung eines elektromagnetischen Feldes oder der Konduktanz eines elektrischen Stromes erhalten wird.

10. Verfahren nach irgendeinem der vorhergehenden Ansprüche 1 bis 9 für die Quantifizierung des Volumens von einem oder mehreren Niederschlägen über einer definierten Oberfläche des festen Trägers, **dadurch gekennzeichnet, dass** Bilder von der besagten definierten Oberfläche, die einen oder mehrere Niederschläge enthalten und die verschiedenen Sichten entsprechen, wobei die besagten Bilder analoge Informationen enthalten, von einer oder von mehreren Kameras nach einer Beleuchtung durch eine oder mehrere Beleuchtungsquellen aufgenommen werden, die zwar räumlich gemäß einem vorbestimmtem Muster relativ zueinander angeordnet sind und **dadurch gekennzeichnet, dass** die entsprechenden analogen Informationen des Bildes der besagten definierten Oberfläche, die den einen oder die mehreren Niederschläge enthält, in eine digitale Form oder in einen Satz digitaler Formen transformiert und umgesetzt werden und mit einem ersten und mit einem zweiten Referenzstandard verglichen werden, um das Volumen des Niederschlages oder der Niederschläge zu bestimmen, die quantifiziert werden sollen.

11. Verfahren nach Anspruch 10, **dadurch gekennzeichnet, dass** der erste Referenzstandard einer digitalen Form oder einem Satz digitaler Formen entspricht, die aus analogen Informationen gewonnen worden sind, die in Bildern enthalten sind, welche auf der Oberfläche des besagten festen Trägers (1) ohne Niederschlag aufgenommen worden sind.

12. Verfahren nach Anspruch 11, **dadurch gekennzeichnet, dass** der zweite Referenzstandard einer digitalen Form oder einem Satz digitaler Formen entspricht, die aus analogen Informationen gewonnen worden sind, die in Bildern enthalten sind, welche auf der Oberfläche des besagten festen Trägers (1) aufgenommen worden sind, die einen oder mehrere Niederschläge mit einem bekannten Volumen enthält.

13. Vorrichtung für die Diagnostik und / oder die Quantifizierung von einer oder von mehreren identischen Zielverbindungen, die aus einer Probe gewonnen worden sind, bestehend aus
- Einfangmolekülen, die auf einer Oberfläche eines festen Trägers gebunden sind, in einer Gruppierung, die eine Dichte von mindestens 20 diskreten Bereichen pro cm² aufweist, wobei ein jeder dieser diskreten Bereiche mit einer Spezies eines Einfangmoleküls verbunden ist,
- einem metallischen Niederschlag, der an einer Stelle der Bindung zwischen besagtem Zielmolekül mit besagtem Einfangmolekül gebildet wird,
- einem Gerät zum Nachweis und / oder zur Quantifizierung eines metallischen Niederschlages, der an einer Stelle der Bindung zwischen der besagten Zielverbindung mit dem Einfangmolekül gebildet worden ist,
- möglicherweise ein Lesegerät für Informationen, die auf dem festen Träger aufgezeichnet sind, und
- ein Computer, der dazu programmiert ist, um:
- möglicherweise diskrete Bereiche zu erkennen, die Einfangmoleküle tragen,
- die Ergebnissee zu sammeln (die aus der Bildung eines metallischen Niederschlages an einer bestimmten Stelle stammen), die von dem Nachweisgerät erhalten werden, und möglicherweise die Informationen, die aus dem besagten Lesegerät erhalten worden sind, und
- die Durchführung einer Diagnostik und / oder einer Quantifizierung der einen oder mehreren Zielverbindungen,
mit der Maßgabe, dass als fester Träger eine Kompaktdiskette (CD) ausgeschlossen wird, wobei die nicht-spaltbaren Einfangmoleküle auf der äußeren Oberfläche der Kompaktdiskette durch konventionelle physikalische Verfahren adressiert werden unter Verwendung mikrolithographischer und/oder mikroverarbeitender Techniken der Inkubation, welche die nicht-spaltbaren Einfangmoleküle an bestimmten Stellen halten, an denen sie fixiert sind.

## Revendications

1. Procédé d'identification et/ou de quantification d'un composé cible obtenu à partir d'un échantillon, de préférence un échantillon biologique, comprenant les étapes suivantes :
- mettre en contact le composé cible avec une molécule de capture afin de permettre une liaison spécifique entre ledit composé cible et une molécule de capture, ladite molécule de capture étant fixée sur une surface d'un support solide conformément à un réseau comprenant une densité d'au moins 20 régions discrètes par cm², chacune desdites régions discrètes étant fixée à une espèce de molécules de capture,
- exécuter une réaction entraînant la formation d'un précipité formé à l'emplacement de ladite liaison et obtenu par le précipité d'un composé métallique sur le composé cible lié,
- déterminer l'éventuelle formation et/ou présence d'un/de précipité(s) dans une/des région(s) discrète(s), et à mettre en corrélation la formation et/ou la présence du/des précipité(s) au niveau de la/des régions(s) discrète(s) avec l'identification et/ou une quantification dudit composé cible, à condition qu'en tant que support solide un disque compact, dans lequel les molécules de capture non-clivables sur la surface externe du disque compact sont traitées par des procédés physiques traditionnels utilisant des techniques d'incubation microlithographique et/ou de micro-usinage qui maintiendront les molécules de capture non-clivables à certains emplacements où elles seront fixées, soit exclu.

2. Procédé selon la revendication 1, **caractérisé en ce que** ledit composé métallique est un composé métallique magnétique.

3. Procédé selon la revendication 1 ou la revendication 2, **caractérisé en ce que** la réaction entraînant la formation du précipité est une réduction d'un métal en présence d'une enzyme.

4. Procédé selon la revendication 1 ou la revendication 2, **caractérisé en ce que** la réaction entraînant la formation du précipité est une réduction chimique d'argent en présence de particules d'or colloïdal couplées au composé cible lié.

5. Procédé selon l'une quelconque des revendications précédentes, **caractérisé en ce que** la liaison spécifique entre le composé cible et sa molécule de capture correspondante est une hybridation entre deux séquences de nucléotides.

6. Procédé selon l'une quelconque des revendications 1 à 4, **caractérisé en ce que** la liaison entre le composé cible et sa molécule de capture correspondante est une réaction entre une structure antigénique et son anticorps correspondant ou une partie hypervariable de celui-ci.

7. Procédé selon l'une quelconque des revendications précédentes, **caractérisé en ce que** la liaison entre le composé cible et sa molécule de capture correspondante est une réaction entre un récepteur et son ligand correspondant.

8. Procédé selon l'une quelconque des revendications précédentes, **caractérisé en ce que** l'éventuelle présence d'un précipité est obtenue par réflexion, absorption ou diffusion d'un faisceau lumineux, de préférence un faisceau laser, sur ledit précipité.

9. Procédé selon l'une quelconque des revendications précédentes, **caractérisé en ce que** la présence d'un précipité dans une région discrète est obtenue par variation d'un champ électromagnétique ou par la conductance d'un courant électrique.

10. Procédé selon l'une quelconque des revendications précédentes 1 à 9, pour la quantification de volume d'un ou de plusieurs précipité(s) sur une surface définie du support solide, **caractérisé en ce que** des images de ladite surface définie contenant un ou plusieurs précipité(s) et correspondant à différentes vues, lesdites images contenant des informations analogiques, sont prises par un ou plusieurs appareil(s) de prise de vues lors de l'éclairage par une ou plusieurs source(s) lumineuse(s), disposées dans l'espace les unes par rapport aux autres conformément à un modèle prédéterminé et **caractérisé en ce que** les informations analogiques d'images correspondantes de ladite surface définie contenant ledit/lesdits précipité(s) sont transformées et converties au format numérique ou en un ensemble de formats numériques puis comparées à une première et à une deuxième normes de référence afin de déterminer le volume du/des précipité(s) à quantifier.

11. Procédé selon la revendication 10, **caractérisé en ce que** la première norme de référence correspond à un format numérique ou à un ensemble de formats numériques obtenu à partir des informations analogiques contenues dans des images prises sur la surface dudit support solide (1) sans précipité.

12. Procédé selon la revendication 11, **caractérisé en ce que** la deuxième norme de référence correspond à un format numérique ou à un ensemble de formats numériques obtenu à partir des informations analogiques contenues dans des images prises sur la surface dudit support solide (1) contenant un/des précipité(s) d'un volume connu.

13. Appareil de diagnostic et/ou de quantification d'un ou de plusieurs composé(s) cible(s) identique(s) obtenu(s) à partir d'un échantillon, qui comprend :
- des molécules de capture fixées sur une surface d'un support solide conformément à un réseau comprenant une densité d'au moins 20 régions discrètes par cm², chacune desdites régions discrètes étant fixée à une espèce de molécule de capture ;
- un précipité métallique formé à un emplacement de la liaison entre ledit composé cible et ladite molécule de capture,
- un dispositif de détection et/ou de quantification d'un précipité métallique formé à un emplacement de la liaison entre ledit composé cible et ladite molécule de capture,
- éventuellement un dispositif de lecture d'information(s) enregistrée(s) sur ledit support solide, et
- un ordinateur programmé pour :
- éventuellement reconnaître des régions discrètes comportant des molécules de capture,
- collecter les résultats (résultant de la formation d'un précipité métallique à un emplacement spécifique) obtenus à partir dudit dispositif de détection, et éventuellement l'/les information(s) obtenue(s) à partir dudit dispositif de lecture, et
- effectuer un diagnostic et/ou une quantification dudit/desdits composé(s) cible(s), à condition qu'en tant que support solide un disque compact, dans lequel les molécules de capture non-clivables sur la surface externe du disque compact sont traitées par des procédés physiques traditionnels utilisant des techniques d'incubation microlithographique et/ou de micro-usinage qui maintiendront les molécules de capture non-clivables à certains emplacements où elles seront fixées, soit exclu.
